# NEUE EUROPÄISCHE PATENTSCHRIFT

(11) **EP 1 746 101 B2**
(45) Veröffentlichungstag und Bekanntmachung des Hinweises auf die Entscheidung über den Einspruch: **14.05.2014**
(45) Hinweis auf die Patenterteilung: 06.08.2008
(21) Anmeldenummer: 05090214.7
(22) Anmeldetag: 21.07.2005
(51) Int. Cl.: C07J 53/00

(54) **Verfahren zur Herstellung von 3-Oxo-pregn-4-en-21,17-carbolactonen durch die metallfreie Oxidation von 17-(3-hydroxypropyl)-3,17-dihydroxyandrostanen**
Process for the production of Oxo-pregn-4-en-21,17-carbolactonen by metal free oxidation of 17-(3-hydroxypropyl)-3,17-dihydroxyandrostanes
Procédé pour la production des Oxo-pregn-4-en-21,17-carbolactones par l'oxidation des 17-(3-hydroxypropyl)-3,17-dihydroxyandrostanes sans utilisation des métaux

(43) Veröffentlichungstag der Anmeldung: 24.01.2007
(73) Patentinhaber: Bayer Pharma Aktiengesellschaft, 13353 Berlin (DE)
(72) Erfinder: Seilz, Carsten, 59199 Bönen (DE); Seba, Hartmut, 59423 Unna (DE)
(74) Vertreter: BIP Patents

(56) Entgegenhaltungen:
- WO-A-98/06738
- US-A1- 2004 220 158
- BITTLER D ET AL: "SYNTHESIS OF SPIRORENONE - A NOVEL HIGHLY ACTIVE ALDOSTERONE ANTAGONIST" ANGEWANDTE CHEMIE. INTERNATIONAL EDITION, WILEY VCH VERLAG, WEINHEIM, DE, Bd. 21, Nr. 9, 1982, Seiten 696-697, XP002047531 ISSN: 1433-7851
- NICKISCH K ET AL: "ALDOSTERONE ANTAGONISTS 2. NEW 7-ALPHA ACETYLTHIO-15 16-METHYLENESPIROLACTONES" JOURNAL OF MEDICINAL CHEMISTRY, Bd. 30, Nr. 8, 1987, Seiten 1403-1409, XP002367543 ISSN: 0022-2623
- ZHANG ET AL: "Asymmetric Dihydroxylation-Haloetherification Strategy for the Synthesis of Tetrahydrofuran-Containing Acetogenins" JOURNAL OF ORGANIC CHEMISTRY, AMERICAN CHEMICAL SOCIETY. EASTON, US, Bd. 63, Nr. 6, 1998, Seiten 2049-2052, XP002104971 ISSN: 0022-3263
- ANELLI P L ET AL: "OXIDATION OF DIOLS WITH ALKALI HYPOCHLORITES CATALYZED BY OXAMMONIUM SALTS UNDER TWO-PHASE CONDITIONS" JOURNAL OF ORGANIC CHEMISTRY, AMERICAN CHEMICAL SOCIETY. EASTON, US, Bd. 54, Nr. 12, 9. Juni 1989 (1989-06-09), Seiten 2970-2972, XP000025425 ISSN: 0022-3263

## Beschreibung

Die vorliegende Erfindung betrifft Verfahren zur Herstellung von 3-Oxo-pregn-4-en-21,17-carbolactonen, insbesondere Verfahren zur Herstellung von 3-Oxo-17α-pregnan-21,17-carbolactonen sowie 3-Oxo-17α-pregn-4-en-21,17-carbolactonen. Des weiteren betrifft die Erfindung das Dichlormethan-Hemisolvat von 6β, 7β; 15β, 16β-dimethylen-3-oxo-17α-pregnan-5β-ol-21,17-carbolacton.

Beispiele für pharmakologisch wirksame Steroid-21,17-carbolactonen sind Eplerenon (9α, 11α-Epoxy-7α-methoxycarbonyl-3-oxo-17α-pregn-4-en-21,17-carbolacton), Drospirenon (6β, 7β; 15β, 16β-dimethylen-3-oxo-17α-pregn-4-en-21,17-carbolacton, Spironolacton (7α-Acetylthio-3-oxo-17α-pregn-4-en-21,17-carbolacton, Canrenone (3-oxo-17α-pregna-4,6-dien-21,17-carbolacton), Prorenon (6β,7β-Methylen-3-oxo-17α-pregna-4,6-dien-21,17-carbolacton).
Der Aufbau des Steroid-21,17-Spirolactons kann durch Oxidation des entsprechenden 17-Hydroxy-17-(3-Hydroxypropyl)steroids 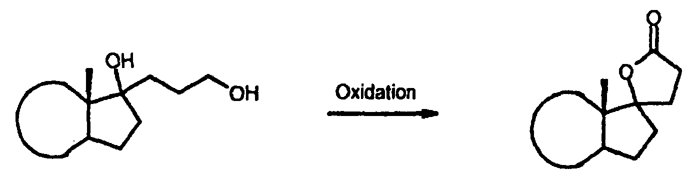 mit geeigneten Oxidationsmitteln wie Chromsäure (Sam et al. J. Med. Chem. 1995, 38, 4518-4528), Pyridiniumchlorochromat (EP 075189), Pyridiniumdichromat (Bittler et al; Angew. Chem. 1982, 94, 718-719; Nickisch et al. Liebigs Ann. Chem. 1988, 579-584), oder Kaliumbromat in Gegenwart eines Rutheniumkatalysators (EP 918791) erfolgen. Nachteilig bei den Oxidationsverfahren des Standes der Technik mit Chrom(VI)-Derivaten ist die deutlich ausgeprägte Nebenproduktbildung durch eine Vielzahl von Nebenreaktionen, wodurch die Isolierung des reinen Produktes erschwert, und die Ausbeute vermindert werden. Das Nebenproduktprofil wird zwar durch die Rutheniumkatalysierte Oxidation (EP 918791) verbessert und damit auch die Ausbeute erhöht. Allerdings ist die Verwendung von Übergangsmetallen in der Produktion pharmazeutischer Wirkstoffe generell mit dem Nachteil behaftet, dass die Entfernung von Schwermetallspuren stets mit einem erhöhten Aufwand verbunden ist. Darüber hinaus fallen in der Produktion große Mengen an schwermetallhaltigen Abfällen an, die nur aufwändig und teuer entsorgt werden können.

Die Aufgabe der vorliegenden Erfindung besteht daher darin, ein alternatives Verfahren zur Herstellung von 3-Oxo-pregn-4-en-21,17-carbolactonen aus den entsprechenden 17-(3-hydroxypropyl)-3,17-dihydroxyandrostanen zur Verfügung zu stellen, das ermöglicht die Zielverbindungen mit einer höheren Ausbeute und Reinheit herzustellen.

Diese Aufgabe wurde erfindungsgemäß durch ein Verfahren gelöst, das folgende Schritte umfasst:
a) die Umsetzung von Verbindungen der allgemeinen Formel I 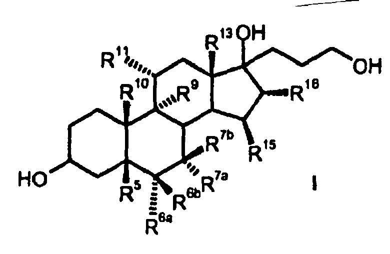 worin
   - R⁵: hydroxy
   - R^{6a}: Wasserstoff, oder zusammen mit R^{7a} eine -CH₂-Gruppe;
   - R^{6b}: Wasserstoff, zusammen mit R^{7b} eine -CH₂-Gruppe oder eine Doppelbindung;
   - R^{7a}: Wasserstoff, C₁-C₄-Alkyl, C₁-C₄-Alkoxycarbonyl, C₁-C₄-Thioacyl oder zusammen mit R^{6a} eine -CH₂-Gruppe
   - R^{7b}: Wasserstoff, oder zusammen mit R^{6b} eine -CH₂-Gruppe
   - R⁹: Wasserstoff, zusammen mit R¹¹ eine Doppelbindung oder zusammen mit R¹¹ eine Epoxygruppe -O-;
   - R¹⁰: Wasserstoff, Methyl, Ethyl;
   - R¹¹: Wasserstoff, zusammen mit R⁹ eine Doppelbindung oder zusammen mit R⁹ eine Epoxygruppe -O-;
   - R¹³: Wasserstoff, Methyl, Ethyl;
   - R¹⁵: Wasserstoff, C₁-C₄-Alkyl, zusammen mit R¹⁶ eine -CH₂-Gruppe oder eine Doppelbindung;
   - R¹⁶: Wasserstoff, zusammen mit R¹⁵ eine -CH₂-Gruppe oder eine Doppelbindung,
   mit mindestens 3 moleq. eines organischen oder anorganischen Hypochlorits als Oxidationsmittel in Gegenwart von katalytischen Mengen eines 2,2,6,6-Tetramethylpiperidin-N-Oxid-Derivates bei einem pH-Wert von mindestens 8,0 in einem zweiphasigen Dichlormethan-Wasser-Gemisch
   zu den Verbindungen der Formel 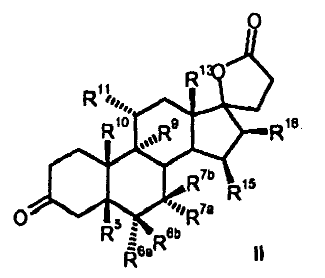
b) Isolierung der Verbindung der Formel II,
c) anschließende Wassereliminierung bei pH < 5, gegebenenfalls in Gegenwart einer Säure.

Metallfreie Oxidationen von Alkoholen zu den entsprechenden Aldehyden, Ketonen, Carbonsäuren, Lactolen, Lactonen sind in dem Übersichtsartikel W. Adam et al, Chem. Rev. 2001, 101, 3499 - 3548 zusammengefasst. Metallfreie Oxidationen in Gegenwart von 2,2,6,6-Tetramethylpiperidin-N-Oxid (TEMPO) sind beschrieben von van Bekkum et al. in Synthesis 1996, 1153-1174.

Primäre Alkohole können mit Natriumbromit (NaBrO₂) oder Kalciumhypochlorit [Ca(OCl)₂] in Gegenwart von TEMPO-Derivaten zu Aldehyden oxidiert werden [S. Torii et al J. Org. Chem. 1990, 55, 462-466]. Natriumhypochlorit (NaOCl) kann auch als Oxidationsmittel verwendet werden (Org. Synth. 69, 212).
Die Oxidation von sekundären Alkoholen zu Ketonen und insbesondere die Oxidation von primären Alkoholen zu Carbonsäuren (bzw. bei geeigneten Diolen zu Lactonen erfordert einen Cokatalysator (P. L. Anelli et al, J. Org. Chem. 1987, 52, 2559-2562). Als Cokatalysator wird ein Bromid (in der Regel KBr oder NaBr) verwendet. Die Zugabe von Bromidionen kann selbst bei der Oxidation von primären Alkoholen zu Aldehyden sinnvoll sein (P. L. Anelli et al, J. Org. Chem. 1987, 52, 2559-2562).
Nachteilig an der Verwendung von Bromiden als Cokatalysator ist die Gefahr der Bildung von bromhaltigen Nebenprodukten unter den oxidativen Bedingungen. Diese Oxidationsmethode ist besonders für die Oxidation von primären Alkoholen zu den entsprechenden Aldehyden geeignet.
Ohne Bromidzusatz bedarf die TEMPO-katalysierte Oxidation von sekundären Alkoholen zu den entsprechenden Ketonen hoher Überschüsse an dem Hypochlorit [3-4 moleq. Ca(OCl)₂ also 6-8 moleq. OCl⁻; S. Tori et al J. Org. Chem. 1990, 55, 462-466].
Die oxidative Lactonisierung von 1,4-Diolen läuft mehrstufig über den Aldehyd ab, welcher zunächst intermediär das Lactol bildet, dessen quasi-sekundäre Hydroxygruppe dann weiteroxidiert werden muss. Daher erfordert die oxidative Lactonisierung von 1,4-Diolen noch härtere Bedingungen (mindestens equimolare Mengen an dem TEMPO-Derivat (J. M. Bobbitt et al, J. Org. Chem. 1991, 56, 6110-6114) oder andere Oxidationsmittel in Verbindung mit erhöhten Mengen des TEMPO-Katalysators (J. Einhorn, J. Org. Chem. 1996, 61, 7452-7454; in Gegenwart eines Bromidzusatzes: S. D. Rychnovsky, J. Org. Chem. 1999, 64, 310-312; in Gegenwart in situ aus dem Oxidationsmittel Natriumbromit erzeugter Bromidionen: S. Torii, J. Org. Chem. 1990, 55, 462-466).

Im Angesicht des Standes der Technik ist es daher überraschend gewesen, dass sich die oxidative Lactonisierung am D-Ring und die Oxidation der sekundäre 3-Hydroxygruppe der 17-(3-hydroxypropyl)-3,17-dihydroxyandrostanen der allgemeinen Formel I (insgesamt drei Oxidationsstufen) gleichzeitig unter milden Bedingungen in Gegenwart von katalytischen Mengen an TEMPO-Derivaten erfolgreich durchführen lässt. Des weiteren ist es überraschend gewesen, dass das erfindungsgemäße Verfahren mit nur 1,0 bis 2,0 Equivalenten Hypochlorit pro Oxidationsstufe, also insgesamt 3,0 bis 6,0 moleq Hypochlorit ganz ohne die cokatalytischen Bromidzusätze durchgeführt werden kann.

Das erfindungsgemäße Verfahren wird mit insgesamt mindestens 3 moleq. Alkalihypochlorit, organisches Hypochlorit bzw. mind. 1,5 moleq. Erdalkalihypochlorit als Oxidationsmittel; bevorzugt mit 3-6 moleq. Alkali-, bzw. 1,5-3 moleq. Erdalkalihypochlorit besonders bevorzugt 3-4 moleq. Alkali-, bzw. 1,5-2 moleq. Erdalkalihypochlorit durchgeführt.
Bevorzugt wird die Konzentration der wässrigen Hypochlorit-Lösung während der Oxidation so eingestellt, dass sie 0,8 bis 1,1 mol Hypochlorit/kg beträgt.
Bevorzugt werden Natriumhypochlorit, Kaliumhypochlorit, Calciumhypochlorit oder tert-Butylhypochlorit als Oxidationsmittel verwendet.
Die 2,2,6,6-Tetramethylpiperidin-N-Oxid-Derivate (TEMPO-Derivate) werden in katalytischen Mengen eingesetzt, wobei die Menge bevorzugt 1-5 mol% besonders bevorzugt 1-1,5 mol% beträgt.
Geeignete TEMPO-Derivate sind u.a. das 2,2,6,6-Tetramethylpiperidin-N-Oxid (TEMPO), das 4-Methoxy-2,2,6,6-Tetramethylpiperidin-N-Oxid (4-MeO-TEMPO) sowie das 4-Benzyloxy-2,2,6,6-Tetramethylpiperidin-N-Oxid (4-BnO-TEMPO).
Bevorzugt gemäß vorliegender Erfindung wird TEMPO verwendet, besonders bevorzugt in einer Menge von 1-5 mol%, ganz besonders bevorzugt 1-1,5 mol%.
Die Oxidation wird in einem organischen Lösungsmittel oder in einem zweiphasigen Lösungsmittel-Wasser-Gemisch durchgeführt, wobei das Lösungsmittel so gewählt wird, dass sich darin sowohl das TEMPO-Derivat als die Verbindungen der Formel I gut lösen.
Bevorzugt wird die Reaktion in einem Zweiphasen-System durchgeführt. Ganz bevorzugt wird das erfindungsgemäße Verfahren in Dichlormethan-Wasser-Gemisch durchgeführt.

Die Oxidation wird erfindungsgemäß bei einer Temperatur von 0 bis 20°C, bevorzugt bei 10-20°C durchgeführt.
Der pH-Wert der Reaktionslösung soll während der Oxidation mindestens 8,0; bevorzugt 8,5 bis 10,0; besonders bevorzugt 9,0 bis 9,5 betragen.
Zweckmäßig kann der pH-Wert mit einer geeigneten Brönsted-Säure, wie organischen (z. B. Essigsäure) oder anorganischen Säuren (HCl, H₂SO₄, H₃PO₄), bzw. sauere Salzen von mehrwertigen Säuren (Hydrogencarbonate, Hydrogensulfate, Hydrogenphosphate etc) eingestellt werden. Bevorzugt werden Alkalihydrogencarbonate, besonders bevorzugt Kaliumhydrogencarbonat verwendet.
Die Oxidationsreaktion wird durch Zugabe eines Reduktionsmittels zur Vernichtung des überschüssigen Hypochlorit-Reagenzes abgebrochen. Hierfür ist jedes dem Fachmann bekannte Reduktionsmittel mit entsprechendem Redoxpotential geeignet. Bevorzugt gemäß vorliegender Erfindung wird eine wässrige Alkalihydrogensulfit-Lösung verwendet. Besonders bevorzugt wird Natrium- oder Kaliumhydrogensulfit (NaHSO₃ oder KHSO₃), die wässrige Lösung von Natrium- oder Kaliumdisulfit (Na₂S₂O₅ oder K₂S₂O₅) verwendet.
Wird im Reaktionsgemisch das überschüssige Hypochlorit-Reagenz unter Zusatz einer Base oder eines basischen Puffers bei pH > 5 vernichtet, so können die 3-Oxo-pregnan-21,17-carbolactone der Formel II isoliert werden. Das Beenden der Oxidationsreaktion bei einem pH-Wert von größer als 5 ermöglicht die gezielte Herstellung von Verbindungen der Formel II.
Da im Falle R⁵ = OH die Löslichkeit der Verbindungen der Formel II im Vergleich zu Verbindungen der Formel III in organischen Lösungsmitteln niedriger ist, bietet die gezielte Isolierung der Verbindungen der Formel II als Zwischenstufe auf dem Weg zu Verbindungen der Formel III den besonderen Vorteil der Möglichkeit einer effektiveren Reinigung (z.B. durch Kristallisation). Die gereinigten Zwischenstufen können nach den in der Literatur bekannten Methoden mit einer geeigneten Säure (wie z. B. Schwefelsäure, Salzsäure, para-Toluolsulfonsäure etc) zu Verbindungen der Formel III umgesetzt werden (EP 0918791 ).

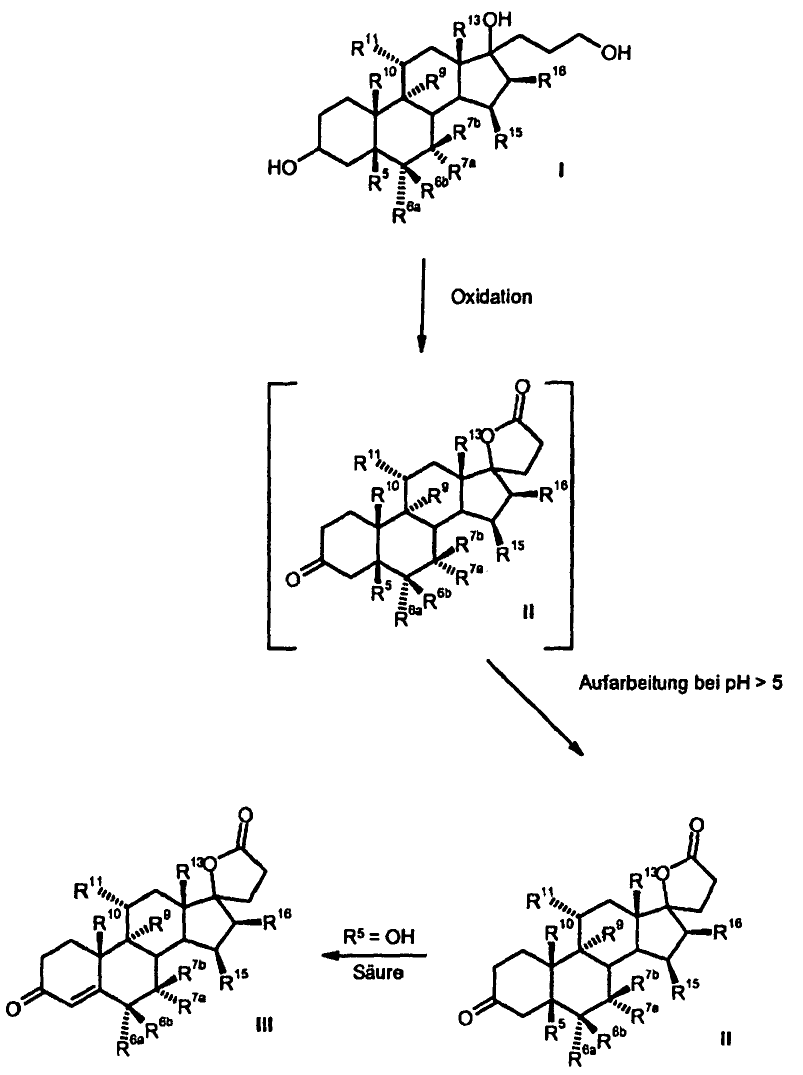

Zur Einstellung des pH-Wertes kann jede geeignete anorganische oder organische Base oder jeder geeignete Puffer bzw. jedes geeignete Puffersystem eingesetzt werden. Bevorzugt wird die Base oder Puffer dem Reaktionsgemisch mit dem Reduktionsmittel gemischt oder parallel hinzugesetzt.
Gemäß vorliegender Erfindung wird bevorzugt Natriumphosphat (Na₃PO₄) als basischer Puffer verwendet.
17-(3-hydroxypropyl)-3,17-dihydroxyandrostanen der allgemeinen Formel I können z.B. ausgehend von den entsprechend substituierten 3-Hydroxy-17-ketoandrostanen durch die Addition von Propargylalkohol an C-17 und anschließende Hydrierung der Dreifachbindung (EP 918791, EP 51143, DE 3026783), oder wie von N. W. Atwater in J. Org. Chem. 1961, 26, 3077 und in US 4,069,219 bzw. in den darin zitierten Dokumenten beschrieben, erhalten werden.
Die entsprechenden 3-Hydroxy-17-ketoandrostane können wiederum von dem entsprechend substituierten 3-Hydroxyandrost-5-en-17-on ausgehend (EP 51143, DE 3026783) hergestellt werden.

Das erfindungsgemäße Verfahren eignet sich besonders für die Herstellung von 3-Oxo-17α-pregn-4-en-21,17-carbolactonen der Formel IIIa, 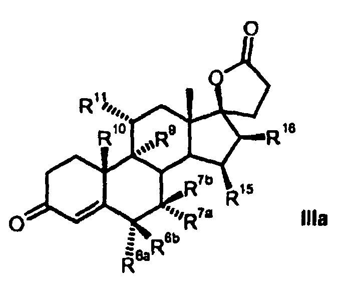 worin
- R^{6a}: Wasserstoff oder zusammen mit R^{7a} eine -CH₂-Gruppe;
- R^{6b}: Wasserstoff, zusammen mit R^{7b} eine -CH₂-Gruppe oder eine Doppelbindung;
- R^{7a}: Wasserstoff, C₁-C₄-Alkoxycarbonyl, C₁-C₄-Thioacyl;
- R^{7b}: Wasserstoff, oder zusammen mit R^{6b} eine -CH₂-Gruppe
- R⁹: Wasserstoff, zusammen mit R¹¹ eine Doppelbindung oder zusammen mit R¹¹ eine Epoxygruppe -O-;
- R¹⁰: Wasserstoff, Methyl;

- R¹¹: Wasserstoff, zusammen mit R⁹ eine Doppelbindung oder zusammen mit R⁹ eine Epoxygruppe -O-;
- R¹⁵: Wasserstoff, zusammen mit R¹⁶ eine -CH₂-Gruppe oder eine Doppelbindung;
- R¹⁶: Wasserstoff, zusammen mit R¹⁵ eine -CH₂-Gruppe oder eine Doppelbindung sein können,
wobei als Ausgangsverbindungen die 17-(3-hydroxypropyl)-3,17-dihydroxyandrostanen der allgemeinen Formel Ia 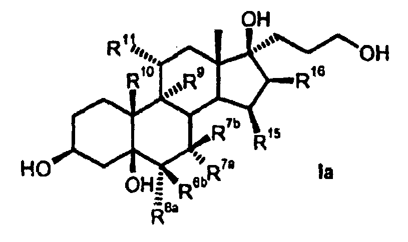 eingesetzt werden.

Ganz besonders eignet sich das erfindungsgemäße Verfahren für die Herstellung der Verbindungen der Formeln IIIa,
worin
- R^{6a}, R^{7a}, R⁹, R¹¹: Wasserstoff;
- R^{6b} und R^{7b}: zusammen eine -CH₂-Gruppe;
- R¹⁰: Methyl;
- R¹⁵ und R¹⁶: zusammen eine -CH₂-Gruppe;
also Verbindungen IIb sowie IIIb, wobei die Verbindung der Formel Ib als Ausgangsverbindung eingesetzt wird.

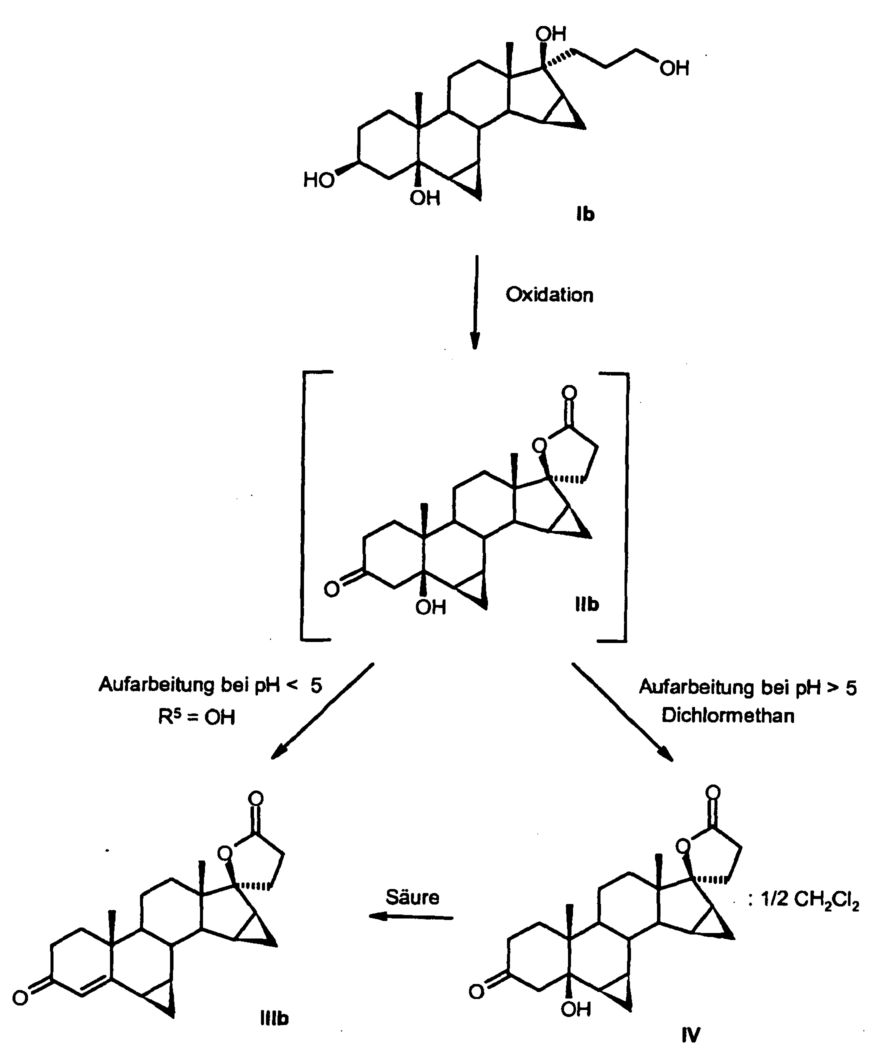

Ein weiterer Aspekt der vorliegenden Erfindung ist das schwerlösliche Dichlormethan-Hemisolvat IV, das sich aus der Verbindung IIb überraschenderweise bildet, wenn das erfindungsgemäße Verfahren in Dichlormethan durchgeführt und basisch bei pH > 5 aufgearbeitet wird. Dieses schwerlösliche Produkt fällt während der Oxidation aus und entzieht sich somit dem Einfluss des Oxidationsmittels und damit möglicher Weiterreaktionen, die zur Bildung von Nebenprodukten führen können.
Das Dichlormethan-Hemisolvat IV zeichnet sich durch einen scharfen und konstanten Schmelzpunkt aus, der bei 121°C liegt, während die Verbindung IIb bei 188°C schmilzt. DSC-Messungen (Differential Scanning Calorimetry) haben gezeigt, dass Verbindung IV bis zum Schmelzpunkt stabil ist.
Nach beendeter Reaktion wird die Fällung der Verbindung IV aus der Reaktionslösung durch Zugabe von einem unpolaren Lösungsmittel, bevorzugt einem Ether, besonders bevorzugt Diisopropylether vervollständigt. Die bei der Oxidation entstehenden unpolaren Oxidations- und Eliminierungsprodukte bleiben im Ether-Dichlormethan-Gemisch weitgehend gelöst, was eine äußerst leichte Isolierung der Verbindung IV in einer hoher Reinheit ermöglicht.
Auf diese Weise wurde Verbindung IV mit einer Ausbeute von 82% erhalten. Das so erhaltene Produkt enthält nicht mehr als 6% steroidale Verunreinigungen, und kann ohne weiteres ohne weitere Aufreinigung nach bekannten Methoden mit einer geeigneten Säure zu Drospirenon IIIb umgesetzt werden (EP 918791). Die Synthesevariante, die über die isolierte Verbindung IV führt, bietet den zusätzlichen Vorteil einer deutlich höheren Gesamtausbeute an IIIb durch einfachere und effektivere Reinigung auf der Endstufe. Die Gesamtausbeute an IIIb liegt bei 77% um ganze 7% höher als nach dem Ru-katalysierten Oxidationsverfahren und anschließender Wassereliminierung und gar um 21% höher als nach dem Eintopfverfahren gemäß EP 075189. (Tab. 1)

**Tab. 1: Vergleich der Ausbeuten des erfindungsgemäßen Verfahrens gegenüber Verfahren des Standes der Technik**

| **Verfahren** | **Ausbeute (% d. Th.)** | | |
|---|---|---|---|
| | **Ib** → **IIb** | **IIb** → **IIIb** | **Gesamt (Ia** → **IIIb)** |
| erfindungsgemäßes Verfahren | 82 (in Form von IV) | 94 | 77 |
| Ru-katalysierte Oxidation nach EP 918791 | 75 | 94 | 70 |
| CrO₃-Oxidation nach EP 075189* | Nicht isoliert | Nicht isoliert | 56 |

| | | | |
|---|---|---|---|
| *siehe Tab. auf S.7 EP 918791 | | | |

Die vorliegende Erfindung wird anhand der nachfolgenden Beispiele näher erläutert, ohne sie darauf einzuschränken.

### Herstellungsverfahren

### Allgemeine Arbeitsvorschrift 1 (AAV1): Synthese von Verbindungen der Formel II

76,9 mmol einer Verbindung der Formel I werden in 135 ml Dichlormethan gelöst bzw. suspendiert. Der Mischung wird bei 15°C zunächst 0,15 g (1 mmol) TEMPO hinzugesetzt. Es erfolgt die Zugabe einer Lösung aus 134 g einer 15,25%-igen wässrigen Natriumhypochlorit-Lösung (230,7 mmol) und 8,20 g (82 mmol) Kaliumhydrogencarbonat in 114 ml Wasser, wobei sich ein pH-Wert von 9,1 einstellt. Nach beendeter Reaktion wird das überschüssige Oxidationsmittel bei 15°C durch Zugabe einer wässrigen Lösung aus 12,5 g (76,5 mmol) Natriumphosphat und 10,6 g (55,8 mmol) Natriumdisulfit (Na₂S₂O₅) und 121 ml Wasser vernichtet.
Das Produkt der Formel II wird aus der organischen Phase isoliert, indem es von der Reaktionslösung durch Zugabe von 240 ml Diisopropylether ausgefällt, 3 h bei 25°C nachgerührt, abfiltriert und getrocknet wird. Alternativ kann das je nach Löslichkeit in Dichlormethan bereits während der Reaktion teilweise ausgefallene Produkt durch Zugabe von Dichlormethan wieder aufgelöst werden, die organische Phase abgetrennt und auf Diisopropylether umdestilliert wird. Das dabei ausgefallene Produkt wird abfiltriert mit 300 ml Wasser gewaschen und getrocknet.

Allgemeine Arbeitsvorschrift 2(AAV2): Synthese von Verbindungen der Formel III ausgehend von Verbindungen der Formel II, worin R5 = OH:
0,1 mol einer Verbindungen der Formel II, worin R⁵ = OH aus AAV1 werden in 65 ml Tetrahydrofuran oder Dioxan suspendiert und durch Zusatz von 5 ml 20%ige Schwefelsäure auf einen pH-Wert von 1 angesäuert. Bei Raumtemperatur wird das Reaktionsgemisch bis zur vollständigen Dehydratisierung nachgerührt.
Die Isolierung des Produktes der Formel III erfolgt durch Ausfällung mittels Zusatz von 90 ml Wasser. Das ausgefallene Produkt wird abfiltriert mit Wasser neutral gewaschen und getrocknet.

### Beispiel 1 6β,7β; 15β, 16β-dimethylen-3-oxo-17α-pregnan-5β-ol-21,17-carbolacton-Dichlormethan-Hemisolvat (IV):

Nach AAV1 werden 30 g (0,0769 mol) 17α-(3-Hydroxypropyl)-6β,7β;15β,16β-dimethylen-androstan-3β,5β,17β-triol umgesetzt.
Während der Reaktion fällt das Produkt 6β,7β;15β,16β-dimethylen-3-oxo-17α-pregnan-5β-ol-21,17-carboiacton in Form dessen Dichlormethan-Hemisolvats aus. Nach Zerstörung des überschüssigen Oxidationsmittels und Aufarbeitung nach AAV1 werden 27 g 6β, 7β; 15β, 16β-dimethylen-3-oxo-17α-pregnan-5β-ol-21,17-carbolacton- Dichlormethan-Hemisolvat (0,0630 mol) = 82 % d. Th isoliert.
[α]_{D}²⁰=-61° (c = 1,0; CHCl₃); Schmelzpunkt. = 121 °C;
¹H-NMR (400 MHz, CDCl₃): δ = 0,52 (q J= 5,5 Hz, 1H, 21α-H [der 15,16-Methylenbrücke]), 0,68-078 (m, 2H, 20-H [der 6,7-Methylenbrücke]), 0,89-0,97 (m, 1 H, 6-H), 0,93 (s, 3H, 19-H), 0,99 (s, 3H, 18-H), 1,19-1,52 (m, 7H,), 1,54-1,85 (m, 6H,), 1,92 (dd J= 3,8 u. 11,8 Hz, 1H, 14-H), 2,06-2,16 (m, 1H, 22-H), 2,17-2,27 (m, 1H, 2α-H), 2,32-2,69 (m, 5H,), 2,96 (d J= 15,6 Hz, 1 H, 4α-H), 5,30 (s , 1 H, CH₂Cl₂). ¹³C-NMR (400 MHz, CDCl₃):δ = 9,97 (CH₂, C-21), 11,63 (CH₂, C-20), 16,74 (CH, C-15), 16,79 (CH, C-7), 17,29 (CH₃, C-19), 19,83 (CH₃, C-18), 21,75 (CH₂, C-11), 24,31 (CH, C-16), 24,76 (CH, C-6), 29,35 (CH₂, C-23), 30,70 (CH₂, C-22), 33,96 (CH, C-8), 34,47 (CH₂, C-1), 36,26 (CH₂, C-2), 37,31 (CH₂, C-12), 40,25 (C, C-10), 41,81 (C, C-13), 47,59 (CH, C-9), 52,18 (CH, C-14), 53,44 (CH₂Cl₂), 53,48 (CH₂, C-4), 75,57 (C, C-5), 96,24 (C, C-17), 176,63 (C, C-24), 210,56 (C, C-3).
MS (El, 70eV) m/e = 384 (M^{+.}); m/e = 366 (M^{+.}-H₂O); m/e =314 (M^{+.}-C₄H₆O); m/e = 111 (C₇H₁₁O^{+.}); m/e = 91 (C₆H₁₁O^{+.}); m/e = 55 (C₃H₃O^{+.}); m/e =43 (C₂H₃O^{+.}).
IR: ϑ=3483 cm⁻¹ (OH); ϑ=1757 cm⁻¹ (C=O, Lacton); ϑ=1708 cm⁻¹ (C=O); ϑ=1200 cm⁻¹ (O-C=O); ϑ=1011 cm⁻¹ (C-O)

## Patentansprüche

1. Verfahren zur Herstellung von 3-Oxo-pregn-4-en-21,17-carbolactonen der Formel III 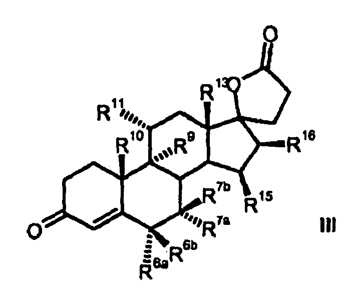 worin
R^{6a} Wasserstoff, oder zusammen mit R^{7a} eine -CH₂-Gruppe;
R^{6b} Wasserstoff, zusammen mit R^{7b} eine -CH₂-Gruppe oder eine Doppelbindung;
R^{7a} Wasserstoff, C₁-C₄-Alkyl, C₁-C₄-Alkoxycarbonyl, C₁-C₄-Thioacyloder zusammen mit R^{6a} eine -CH₂-Gruppe
R^{7b} Wasserstoff, oder zusammen mit R^{6b} eine -CH₂-Gruppe
R⁹ Wasserstoff, zusammen mit R¹¹ eine Doppelbindung oder zusammen mit R¹¹ eine Epoxygruppe -O-;
R¹⁰ Wasserstoff, Methyl, Ethyl;
R¹¹ Wasserstoff, zusammen mit R⁹ eine Doppelbindung oder zusammen mit R⁹ eine Epoxygruppe -O-;
R¹³ Wasserstoff, Methyl, Ethyl;
R¹⁵ Wasserstoff, C₁-C₄-Alkyl, zusammen mit R¹⁶ eine -CH₂-Gruppe oder eine Doppelbindung;
R¹⁶ Wasserstoff, zusammen mit R¹⁵ eine -CH₂-Gruppe oder eine Doppelbindung
umfassend folgende Schritte:
a) die Umsetzung von Verbindungen der allgemeinen Formel I 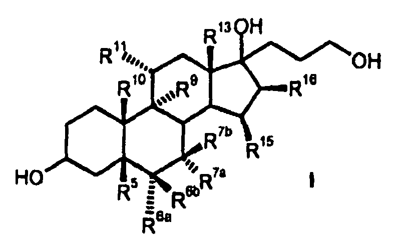 worin
R⁵ Hydroxy;
die Reste R^{6a}, R^{6b} , R^{7a} , R^{7b} , R¹⁰, R¹¹, R¹³ , R¹⁵ , R¹⁶ dieselbe Bedeutung haben wie in Formel III,
mit mindestens 3 moleq. eines organischen oder anorganischen Hypochlorits als Oxidationsmittel in Gegenwart von katalytischen Mengen eines 2,2,6,6-Tetramethylpiperidin-N-Oxid-Derivates bei einem pH-Wert von mindestens 8,0 in einem zweiphasigen Dichlormethan-Wasser-Gemisch
zu den Verbindungen der Formel II 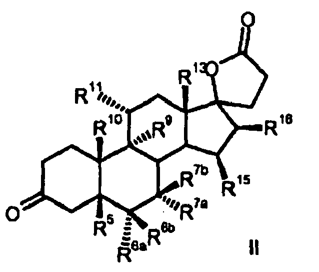
b) Isolierung der Verbindung der Formel II,
c) anschließende Wassereliminierung bei pH < 5, gegebenenfalls in Gegenwart einer Säure.

2. Verfahren nach Anspruch 1 zur Herstellung von 3-Oxo-17α-pregn-4-en-21,17-carbolactonen der Formel IIIa 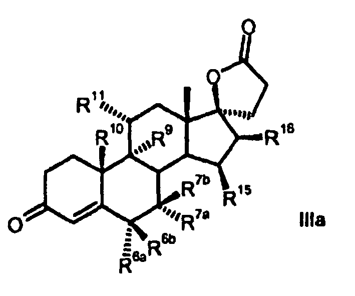 worin
R^{6a} Wasserstoff oder zusammen mit R^{7a} eine -CH₂-Gruppe;
R^{6b} Wasserstoff, zusammen mit R^{7b} eine -CH₂-Gruppe oder eine Doppelbindung;
R^{7a} Wasserstoff, C₁-C₄-Alkoxycarbonyl, C₁-C₄-Thioacyl;
R^{7b} Wasserstoff, oder zusammen mit R^{6b} eine -CH₂-Gruppe
R⁹ Wasserstoff, zusammen mit R¹¹ eine Doppelbindung oder zusammen mit R¹¹ eine Epoxygruppe -O-;
R¹⁰ Wasserstoff, Methyl;
R¹¹ Wasserstoff, zusammen mit R⁹ eine Doppelbindung oder zusammen mit R⁹ eine Epoxygruppe -O-;
R¹⁵ Wasserstoff, zusammen mit R¹⁶ eine -CH₂-Gruppe oder eine Doppelbindung;
R¹⁶ Wasserstoff, zusammen mit R¹⁵ eine -CH₂-Gruppe oder eine Doppelbindung sein können,
**dadurch gekennzeichnet, dass** Verbindungen der Formel Ia 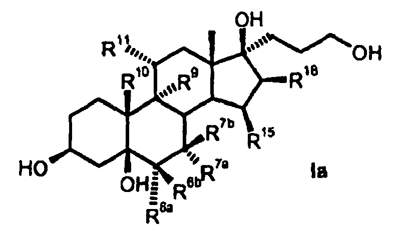 umgesetzt werden.

3. Verfahren nach einem der vorherigen Ansprüche zur Herstellung der Verbindung der Formel IIIb 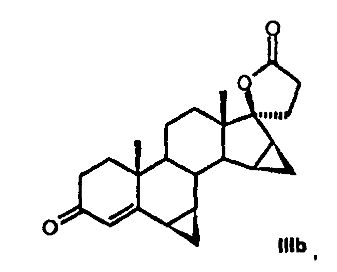 wobei die Verbindung der Formel Ib 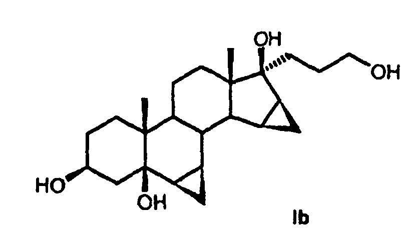 als Ausgangsverbindung eingesetzt wird.

4. Verfahren zur Herstellung des Dichlormethan-Hemisolvats IV: 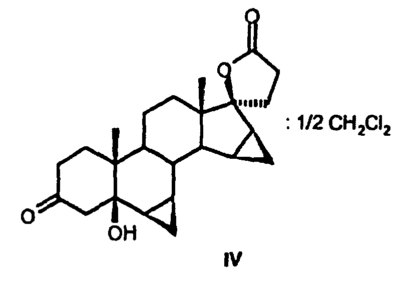 umfassend folgende Schritte:
a) die Umsetzung der Verbindung der Formel Ib 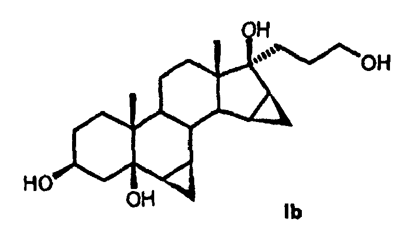 mit mindestens 3 moleq. eines organischen oder anorganischen Hypochlorits als Oxidationsmittel in Gegenwart von katalytischen Mengen eines 2,2,6,6-Tetramethylpiperidin-N-Oxid-Derivates bei einem pH-Wert von mindestens 8,0 in einem zweiphasigen Dichlormethan-Wasser-Gemisch;
b) Isolierung der Verbindung IV.

5. Verfahren nach einem der vorherigen Ansprüche 1 bis 4 **dadurch gekennzeichnet, dass**
1-5 mol% des 2,2,6,6-Tetramethylpiperidin-N-Oxid-Derivates eingesetzt werden.

6. Verfahren nach einem der vorherigen Ansprüche **dadurch gekennzeichnet, dass** 1-1,5 mol% 2,2,6,6-Tetramethylpiperidin-N-Oxid eingesetzt werden.

7. Verfahren nach einem der vorherigen Ansprüchen **dadurch gekennzeichnet, dass** 3-6 moleq. Alkalihypochlorit eingesetzt werden.

8. Verfahren nach einem der vorherigen Ansprüche **dadurch gekennzeichnet, dass** 3-4 moleq. Natriumhypochlorit eingesetzt werden.

9. Verfahren nach einem der vorherigen Ansprüche **dadurch gekennzeichnet, dass** der pH-Wert der Reaktionslösung zwischen 8,5 und 10,0 liegt.

10. Verfahren nach einem der vorherigen Ansprüche **dadurch gekennzeichnet, dass** der pH-Wert der Reaktionslösung mit Kaliumhydrogencarbonat eingestellt wird.

11. Verfahren nach einem der vorherigen Ansprüche **dadurch gekennzeichnet, dass** die Reaktionstemperatur 0 bis 15°C beträgt.

12. Verfahren nach einem der vorherigen Ansprüche **dadurch gekennzeichnet, dass** nach beendeter Oxidationsreaktion dem Reaktionsgemisch ein Reduktionsmittel zur Vernichtung des überschüssigen Hypochlorit-Reagenzes zugesetzt wird.

13. Verfahren nach Anspruch 12 **dadurch gekennzeichnet, dass** das Reduktionsmittel unter Zusatz einer Base oder eines basischen Puffers bei einem pH-Wert von größer als 5 zugesetzt wird.

14. Verfahren nach Anspruch 12 oder 13 **dadurch gekennzeichnet, dass** als Reduktionsmittel eine wässrige Alkalihydrogensulfit-Lösung verwendet wird.

15. Verfahren nach einer der Ansprüche 12 bis 14 **dadurch gekennzeichnet, dass** als Reduktionsmittel Natriumhydrogensulfit oder Kaliumhydrogensulfit in Form der wässrigen Lösung von Natriumdisulfit oder Kaliumdisulfit verwendet wird.

16. Verfahren nach einem der Ansprüche 13 bis 15 **dadurch gekennzeichnet, dass** Natriumphosphat (Na₃PO₄) als Base bzw. basischer Puffer verwendet wird.

17. 6β, 7β; 15β, 16β-dimethylen-3-oxo-17α-pregnan-5β-ol-21,17-carbolacton-Dichlormethan-Hemisolvat.

18. Verfahren zur Herstellung von Drospirenon **dadurch gekennzeichnet, dass** 6β, 7β; 15β, 16β-dimethylen-3-oxo-17α-pregnan-5β-ol-21,17-carbolacton-Dichlor-methan-Hemisolvat (IV) mit einer Säure umgesetzt wird.

19. Verfahren nach Anspruch 18, worin die Säure Schwefelsäure, Salzsäure oder para-Toluolsulfonsäure ist.

## Claims

1. Process for the production of 3-oxopregn-4-ene-21,17-carbolactones of formula III 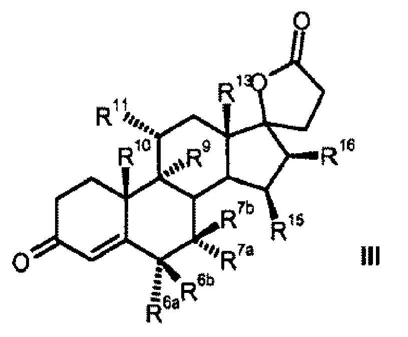 in which
R^{6a} is hydrogen or together with R^{7a} a -CH₂ group;
R^{6b} is hydrogen, together with R^{7b} a -CH₂ group or a double bond;
R^{7a} is hydrogen, C₁-C₄-alkyl, C₁-C₄-alkoxycarbonyl, C₁-C₄-thioacyl or together with R^{6a} a -CH₂ group;
R^{7b} is hydrogen or together with R^{6b} a -CH₂ group;
R⁹ is hydrogen, together with R¹¹ a double bond or together with R¹¹ an epoxy group -O-;
R¹⁰ is hydrogen, methyl, ethyl;
R¹¹ is hydrogen, together with R⁹ a double bond or together with R⁹ an epoxy group -O-;
R¹³ is hydrogen, methyl, ethyl;
R¹⁵ is hydrogen, C₁-C₄-alkyl, together with R¹⁶ a -CH₂ group or a double bond;
R¹⁶ is hydrogen, together with R¹⁵ a -CH₂ group or a double bond;
comprising the following steps:
a) the reaction of compounds of general formula I 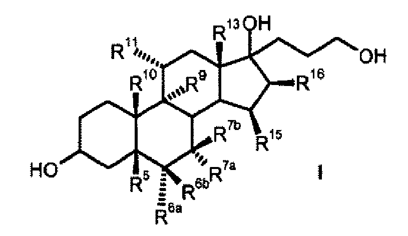 in which
R⁵ is hydroxy;
the radicals R^{6a}, R^{6b}, R^{7a}, R^{7b}, R¹⁰, R¹¹, R¹³, R¹⁵, R¹⁶ have the same meaning as in formula III,
with at least 3 molar equivalents of an organic or inorganic hypochlorite as an oxidizing agent in the presence of catalytic amounts of a 2,2,6,6-tetramethylpiperidine N-oxide derivative at a pH of at least 8 in a two-phase dichloromethane-water mixture,
to form the compounds of formula II 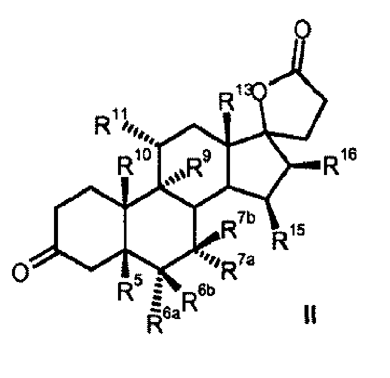
b) isolation of the compound of formula II,
c) subsequent elimination of water at pH < 5, optionally in the presence of an acid.

2. Process according to Claim 1 for the production of 3-oxo-17α-pregn-4-ene-21,17-carbolactones of formula IIIa 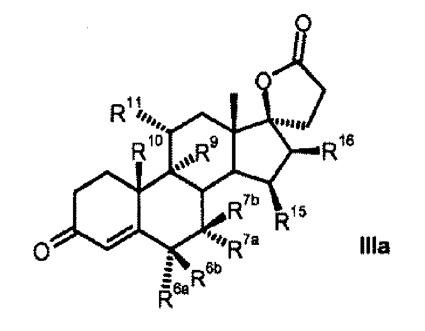 in which
R^{6a} is hydrogen or together with R^{7a} a -CH₂ group;
R^{6b} is hydrogen, together with R^{7b} a -CH₂ group or a double bond;
R^{7a} is hydrogen, C₁-C₄-alkoxycarbonyl, C₁-C₄-thioacyl;
R^{7b} is hydrogen or together with R^{6b} a -CH₂ group;
R⁹ is hydrogen, together with R¹¹ a double bond or together with R¹¹ an epoxy group -O-;
R¹⁰ is hydrogen, methyl;
R¹¹ is hydrogen, together with R⁹ a double bond or together with R⁹ an epoxy group -O-;
R¹⁵ is hydrogen, together with R¹⁶ a -CH₂ group or a double bond;
R¹⁶ is hydrogen, together with R¹⁵ a -CH₂ group or a double bond;
**characterized in that** compounds of formula Ia 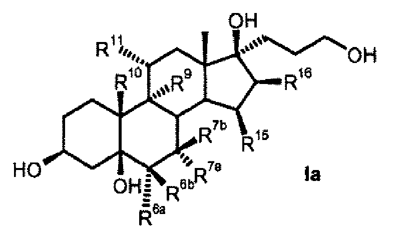 are reacted.

3. Process according to any one of the preceding claims for the production of the compound of formula IIIb 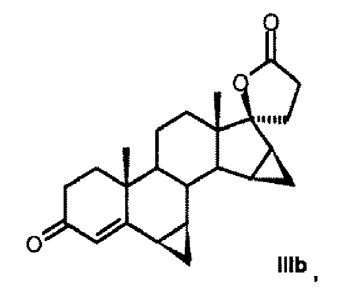 wherein the compound of formula Ib 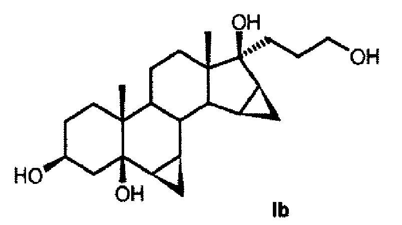 is used as a starting material.

4. Process for the production of the dichloromethane hemisolvate IV: 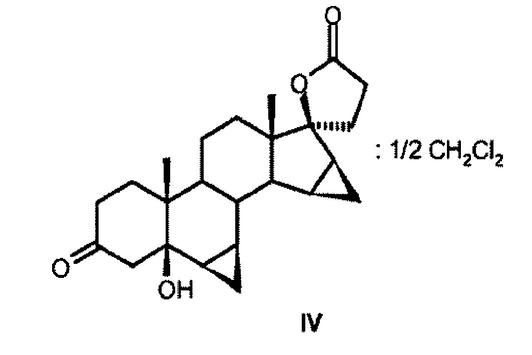 comprising the following steps:
a) the reaction of the compound of formula Ib 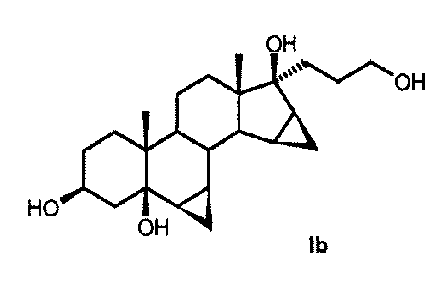 with at least 3 molar equivalents of an organic or inorganic hypochlorite as an oxidizing agent in the presence of catalytic amounts of a 2,2,6,6-tetramethylpiperidine N-oxide derivative at a pH of at least 8 in a two-phase dichloromethane-water mixture;
b) isolation of the compound IV.

5. Process according to any one of the preceding Claims 1 to 4, **characterized in that** 1-5 mol% of the 2,2,6,6-tetramethylpiperidine N-oxide derivative is used.

6. Process according to any one of the preceding claims, **characterized in that** 1-1.5 mol% of 2,2,6,6-tetramethylpiperidine N-oxide is used.

7. Process according to any one of the preceding claims, **characterized in that** 3-6 molar equivalents of alkali hypochlorite are used.

8. Process according to any one of the preceding claims, **characterized in that** 3-4 molar equivalents of sodium hypochlorite are used.

9. Process according to any one of the preceding claims, **characterized in that** the pH of the reaction solution is between 8.5 and 10.0.

10. Process according to any one of the preceding claims, **characterized in that** the pH of the reaction solution is set with potassium bicarbonate.

11. Process according to any one of the preceding claims, **characterized in that** the reaction temperature is 0 to 15°C.

12. Process according to any one of the preceding claims, **characterized in that** after the oxidation reaction has ended a reducing agent for quenching excess hypochlorite reagent is added to the reaction mixture.

13. Process according to Claim 12, **characterized in that** the reducing agent is added with the addition of a base or a basic buffer at a pH of more than 5.

14. Process according to Claim 12 or 13, **characterized in that** an aqueous alkali hydrogen sulphite solution is used as reducing agent.

15. Process according to any one of Claims 12 to 14, **characterized in that** the reducing agent used is sodium hydrogen sulphite or potassium hydrogen sulphite in the form of the aqueous solution of sodium disulphite or potassium disulphite.

16. Process according to any one of Claims 13 to 15, **characterized in that** sodium phosphate (Na₃PO₄) is used as base or basic buffer.

17. 6β,7β;15β,16β-Dimethylene-3-oxo-17α-pregnan-5β-ol-21,17-carbolactone dichloromethane hemisolvate.

18. Process for the production of drospirenone, **characterized in that** 6β,7β;15β,16β-dimethylene-3-oxo-17α-pregnan-5β-ol-21,17-carbolactone dichloromethane hemisolvate (IV) is reacted with an acid.

19. Process according to Claim 18, wherein the acid is sulphuric acid, hydrochloric acid or para-toluenesulphonic acid.

## Revendications

1. Procédé pour la préparation de 3-oxo-prégn-4-ène-21,17-carbolactones de formule III 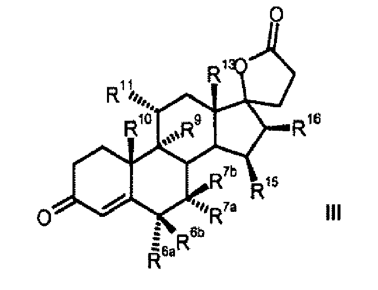 dans laquelle
R^{6a} représente un atome d'hydrogène ou, conjointement avec R^{7a}, un groupe -CH₂- ;
R^{6b} représente un atome d'hydrogène ou, conjointement avec R^{7b}, un groupe -CH₂- ou une double liaison ;
R^{7a} représente un atome d'hydrogène, un groupe alkyle en C₁-C₄, alcoxy(C₁-C₄)carbonyle, thioacyle en C₁-C₄ ou, conjointement avec R^{6a}, un groupe -CH₂- ;
R^{7b} représente un atome d'hydrogène ou, conjointement avec R^{6b}, un groupe -CH₂- ;
R⁹ représente un atome d'hydrogène ou, conjointement avec R¹¹, une double liaison ou, conjointement avec R¹¹, un groupe époxy -O- ;
R¹⁰ représente un atome d'hydrogène, le groupe méthyle ou éthyle ;
R¹¹ représente un atome d'hydrogène ou, conjointement avec R⁹, une double liaison ou, conjointement avec R⁹, un groupe époxy -O- ;
R¹³ représente un atome d'hydrogène, le groupe méthyle ou éthyle ;
R¹⁵ représente un atome d'hydrogène, un groupe alkyle en C₁-C₄ ou, conjointement avec R¹⁶, un groupe -CH₂- ou une double liaison ;
R¹⁶ représente un atome d'hydrogène ou, conjointement avec R¹⁵, un groupe -CH₂- ou une double liaison ;
comprenant les étapes suivantes :
a) la mise en réaction de composés de formule générale I 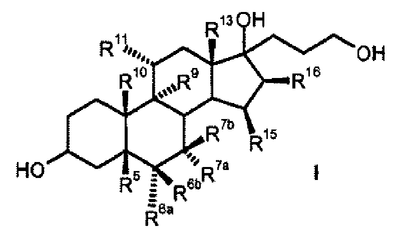 dans laquelle
R⁵ représente un groupe hydroxy ;
les radicaux R^{6a}, R^{6b}, R^{7a}, R^{7b}, R¹⁰, R¹¹, R¹³, R¹⁵, R¹⁶ ont les mêmes significations que dans la formule III,
avec au moins 3 équivalents molaires d'un hypochlorite organique ou inorganique en tant qu'oxydant, en présence de quantités catalytiques d'un dérivé de 2,2,6,6-tétraméthylpipéridine-N-oxyde, à un pH d'au moins 8,0, dans un mélange biphasique de dichlorométhane-eau,
pour aboutir aux composés de formule II 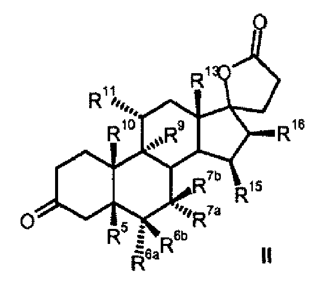
b) l'isolement du composé de formule II,
c) l'élimination subséquente d'eau, à un pH < 5, éventuellement en présence d'un acide.

2. Procédé selon la revendication 1, pour la préparation de 3-oxo-17α-prégn-4-ène-21,17-carbolactones de formule IIIa 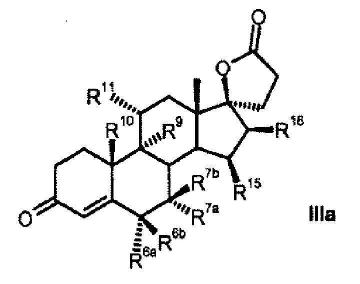 dans laquelle
R^{6a} représente un atome d'hydrogène ou, conjointement avec R^{7a}, un groupe -CH₂- ;
R^{6b} représente un atome d'hydrogène ou, conjointement avec R^{7b}, un groupe -CH₂- ou une double liaison ;
R^{7a} représente un atome d'hydrogène, un groupe alcoxy(C₁-C₄)carbonyle, thioacyle en C₁-C₄ ;
R^{7b} représente un atome d'hydrogène un groupe ou, conjointement avec R^{6b}, un groupe -CH₂- ;
R⁹ représente un atome d'hydrogène ou, conjointement avec R¹¹, une double liaison ou, conjointement avec R¹¹, un groupe époxy -O- ;
R¹⁰ représente un atome d'hydrogène ou le groupe méthyle ;
R¹¹ représente un atome d'hydrogène ou, conjointement avec R⁹, une double liaison ou, conjointement avec R⁹, un groupe époxy -O- ;
R¹⁵ représente un atome d'hydrogène ou, conjointement avec R¹⁶, un groupe -CH₂- ou une double liaison ;
R¹⁶ représente un atome d'hydrogène ou, conjointement avec R¹⁵, un groupe -CH₂- ou une double liaison ;
**caractérisé en ce qu'**on fait réagir des composés de formule Ia

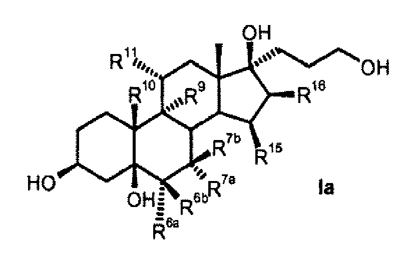

3. Procédé selon l'une quelconque des revendications précédentes, pour la préparation du composé de formule IIIb 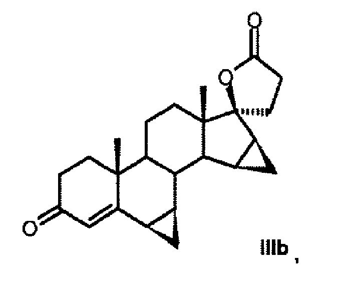 dans lequel on utilise comme produit de départ le composé de formule Ib

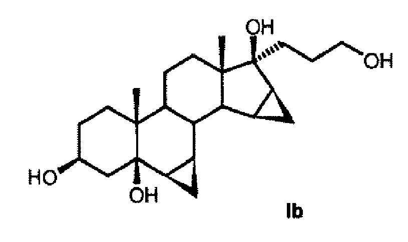

4. Procédé pour la préparation du produit d'hémisolvatation par le dichlorométhane IV : 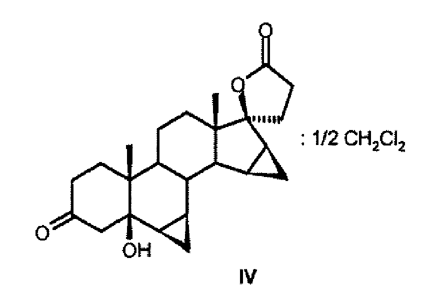 comprenant les étapes suivantes :
a) la mise en réaction du composé de formule Ib 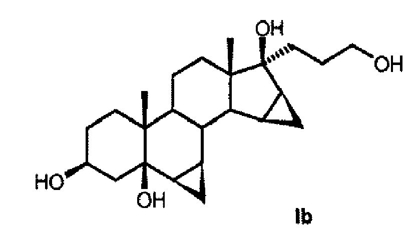 avec au moins 3 équivalents molaires d'un hypochlorite organique ou inorganique en tant qu'oxydant, en présence de quantités catalytiques d'un dérivé de 2,2,6,6-tétraméthylpipéridine-N-oxyde, à un pH d'au moins 8,0, dans un mélange biphasique de dichlorométhane-eau,
b) l'isolement du composé IV.

5. Procédé selon l'une quelconque des précédentes revendications 1 à 4, **caractérisé en ce qu'**on utilise 1-5 % en moles du dérivé de 2,2,6,6-tétraméthylpipéridine-N-oxyde.

6. Procédé selon l'une quelconque des revendications précédentes, **caractérisé en ce qu'**on utilise 1-1,5 % en moles de 2,2,6,6-tétraméthylpipéridine-N-oxyde.

7. Procédé selon l'une quelconque des revendications précédentes, **caractérisé en ce qu'**on utilise 3-6 équivalents molaires d'un hypochlorite de métal alcalin.

8. Procédé selon l'une quelconque des revendications précédentes, **caractérisé en ce qu'**on utilise 3-4 équivalents molaires d'hypochlorite de sodium.

9. Procédé selon l'une quelconque des revendications précédentes, **caractérisé en ce que** pH de la solution réactionnelle est compris entre 8,5 et 10,0.

10. Procédé selon l'une quelconque des revendications précédentes, **caractérisé en ce que** le pH de la solution réactionnelle est ajusté à l'aide d'hydrogénocarbonate de potassium.

11. Procédé selon l'une quelconque des revendications précédentes, **caractérisé en ce que** la température de la réaction est dans la plage de 0 à 15 °C.

12. Procédé selon l'une quelconque des revendications précédentes, **caractérisé en ce qu'**une fois terminée la réaction d'oxydation on ajoute au mélange réactionnel un réducteur pour détruire le réactif hypochlorite en excès.

13. Procédé selon la revendication 12, **caractérisé en ce qu'**on ajoute le réducteur à un pH de plus de 5 par addition d'une base ou d'un tampon basique.

14. Procédé selon la revendication 12 ou 13, **caractérisé en ce qu'**on utilise comme réducteur une solution aqueuse d'hydrogénosulfite de métal alcalin.

15. Procédé selon l'une quelconque des revendications 12 à 14, **caractérisé en ce qu'**on utilise comme réducteur de l'hydrogénosulfite de sodium ou de l'hydrogénosulfite de potassium sous forme de la solution aqueuse de disulfite de sodium ou de disulfite de potassium.

16. Procédé selon l'une quelconque des revendications 13 à 15, **caractérisé en ce qu'**on utilise comme base ou tampon basique du phosphate de sodium (Na₃PO₄).

17. Produit d'hémisolvatation de 6β,7β;15β,16β-diméthylène-3-oxo-17α-prégnan-5β-ol-21,17-carbolactone par le dichlorométhane.

18. Procédé pour la préparation de drospirénone, **caractérisé en ce qu'**on fait réagir avec un acide du produit d'hémisolvatation de 6β,7β;15β,16β-diméthylène-3-oxo-17α-prégnan-5β-ol-21,17-carbolactone par le dichlorométhane (IV).

19. Procédé selon la revendication 18, dans lequel l'acide est l'acide sulfurique, l'acide chlorhydrique ou l'acide p-toluènesulfonique.
